# EUROPEAN PATENT APPLICATION

(11) **EP 2 233 144 A1**
(43) Date of publication of application: **29.09.2010**
(21) Application number: 09701612.5
(22) Date of filing: 14.01.2009
(51) Int. Cl.: A61K 31/722, A61K 9/00, A61K 9/08, A61K 9/14, A61K 47/02, A61K 47/04, A61K 47/32, A61K 47/36, A61P 17/02

(54) **CHITOSAN-CONTAINING COMPOSITIONS AND PROCESS FOR PRODUCTION THEREOF**

(30) Priority: 16.01.2008 JP 2008006843
(71) Applicant: Ebara Corporation, Tokyo 144-8510 (JP)
(72) Inventor: HAYASHI, Masahiro, Tokyo 144-8510 (JP); UMEDA, Isao, Tokyo 144-8510 (JP); MINOWA, Junichi, Tokyo 144-8510 (JP); WATANABE, Kazuo, Tokyo 144-8510 (JP)
(74) Representative: Wagner, Karl H.
(86) International application number: PCT/JP2009/050349
(87) International publication number: WO 2009/090950

(57) **Abstract**

The present invention provides an industrially useful chitosan-containing composition in the form of any of a transparent aqueous solution, gel or dry powder having a neutral to weakly alkaline pH when in the form of an aqueous solution that can be applied to pharmaceutical additives, foods, beverages and cosmetics. A production process of the chitosan-containing composition of the present invention has a step for preparing an acidic chitosan aqueous solution, a step for forming a solid by removing a water-soluble salt from a salt formed by mixing an acidic polymer component into the acidic chitosan aqueous solution, and a step for dissolving the solid in a weakly basic solution.

## Description

### TECHNICAL FIELD

The present invention relates to a chitosan-containing composition that contains chitosan, is in the form of any of a highly transparent solution, gel or dry powder thereof, and is neutral to weakly alkaline when in the form of an aqueous solution, and to a production process thereof.

### BACKGROUND ART

Chitosan is a polysaccharide that contains amino groups, and is known to have been previously used in foods, cosmetics and hair preparations. Moreover, chitosan is a substance that is attracting attention for use in the field of pharmaceuticals, and particularly wound treatment, and as a base of functional preparations.

Although oligosaccharides of glucosamines composing chitosan are known to dissolve in water, the only conventionally known chitosan solutions are acidic solutions in which chitosan is dissolved using an acid such as acetic acid, hydrochloric acid or lactic acid, or acidic solutions thereof. Thus, since chitosan solutions are acidic, when contained in foods or functional foods, only those having a sour taste are able to be manufactured, and since chitosan is in the form of a suspension as a result of not dissolving under neutral or weakly basic conditions, transparent solutions or gels were unable to be produced.

In addition, Non-Patent Document 1 discloses a salt of chitosan, an acidic polymer compound in the form of chondroitin sulfate and dextran sulfate, while Non-Patent Document 2 discloses a salt of chitosan and carrageenan. However, these are not known to be able to be dissolved to form a neutral to weakly alkaline, highly transparent solution, highly viscous solution or gel.
[Non-Patent Document 1] 36th Autumn Annual Meeting of the Society of Chemical Engineers, Japan, Collection of Lecture Abstracts, Lecture No. W2A02, p. 1148
[Non-Patent Document 2] Yamagata University, Faculty of Engineering, Dept. of Polymer Science and Engineering, Collection of 2003 Graduate Research Presentation Abstracts of the Department of Chemistry and Chemical Engineering, C-32

### DISCLOSURE OF THE INVENTION

### [Problems to be Solved by the Invention]

Although chitosan is expected to be used as a food or functional food having a transparent appearance, and particularly as pharmaceutical additive for unstable pharmaceuticals in acidic conditions by using a solution, viscous solution and gel in which chitosan is dissolved under neutral or weakly alkaline conditions, a preparation method and properties of a neutral to weakly alkaline solution, viscous solution and gel in which chitosan is dissolved are unknown.

With the foregoing in view, an object of the present invention is to provide a chitosan-containing composition composed of chitosan, an acidic polymer compound and a carbonate that is industrially useful by being able to be applied to pharmaceutical additives, foods, beverages and cosmetics, and in which is in the form of any of a transparent aqueous solution, gel and dry powder thereof having a neutral to weakly alkaline pH when in the form of an aqueous solution, and a production process thereof.

### [Means for Solving the Problems]

In order to achieve the aforementioned object, the present invention provides a chitosan-containing composition in the form of an aqueous solution, gel or dry powder and containing chitosan, an acidic polymer compound and a carbonate, wherein the pH when in the form of an aqueous solution is within the range of 7.0 to 9.5.

In the chitosan-containing composition of the present invention, the acidic polymer compound is preferably one type or two or more types selected from the group consisting of carrageenan, sodium chondroitin sulfate, sodium dextran sulfate, sodium alginate and polyacrylic acid.

In the chitosan-containing composition of the present invention, the carbonate or bicarbonate is preferably one type or two or more types selected from the group consisting of sodium bicarbonate, ammonium bicarbonate and ammonium carbonate.

In the chitosan-containing composition of the present invention, the chitosan-containing composition is preferably in the form of an aqueous solution.

In the chitosan-containing composition of the present invention, the chitosan-containing composition may also be in the form of a gel.

In the chitosan-containing composition of the present invention, the chitosan-containing composition may also be in the form of a dry powder.

In addition, the present invention provides a process for producing a chitosan-containing composition comprising the steps of:
A) adding acid to chitosan to dissolve it, thereby preparing an acidic chitosan aqueous solution;
B) mixing an acidic polymer compound into the acidic chitosan aqueous solution, thereby forming a water-insoluble salt of the chitosan and the acidic polymer compound, and removing water-soluble salt from the salt, thereby forming a solid; and
C) dissolving the solid in a weakly basic solution either directly or after drying to a solid thereby obtaining a chitosan-containing composition in the form of an aqueous solution or gel.

The process for producing a chitosan-containing composition of the present invention may further comprise a step of drying the chitosan-containing composition in the form of an aqueous solution or gel obtained in the step C, thereby obtaining a chitosan-containing composition in the form of a dry powder.

In the production process of a chitosan-containing composition of the present invention, the acidic polymer compound is preferably one type or two or more types selected from the group consisting of carrageenan, sodium chondroitin sulfate, sodium dextran sulfate, sodium alginate and polyacrylic acid.

In the production process of a chitosan-containing composition of the present invention, the weakly basic solution is preferably an aqueous solution containing one type or two or more types selected from the group consisting of sodium bicarbonate, ammonium bicarbonate and ammonium carbonate.

### [Effects of the Invention]

According to the present invention, a chitosan-containing composition that contains chitosan and is in the form of any of a highly transparent solution having a neutral to weakly alkaline pH when in the form of an aqueous solution, gel or dry powder obtained by drying the solution or gel can be provided, thereby making it possible to provide a product that contains chitosan, has high transparency and has superior sensory properties free from sour taste for use in fields such as foods, beverages, cosmetics and pharmaceuticals.
In addition, the present invention provides a means for incorporating chitosan in compounds having low stability under acidic conditions in fields such as foods, beverages, cosmetics and pharmaceuticals.
In addition, the production process of a chitosan-containing composition of the present invention enables the industrially useful chitosan-containing composition as described above to be easily produced.
In addition, a dry powder of the chitosan-containing composition obtained according to the production process of a chitosan-containing composition of the present invention can be used as a gelling additive in the production of pharmaceuticals.

### BEST MODE FOR CARRYING OUT THE INVENTION

Although the inventors of the present invention initially attempted to make acidic solutions of chitosan neutral to weakly alkaline using various methods, the chitosan ended up precipitating thereby preventing a transparent, neutral to weakly alkaline aqueous solution of chitosan from being obtained. In addition, when an alkaline neutralizer was simply added to neutralize acid being used to dissolve the chitosan, the salt concentration of the solution became high, thereby making the solution undesirable when considering the case of using in fields such as foods, beverages, cosmetics and pharmaceuticals.

Therefore, in order to prevent precipitation, as a result of using a thickener or gelling agent of an acidic polymer compound to form a salt poorly soluble in water, and removing the salt used to dissolve the chitosan by washing with water, it was found that this salt that is poorly soluble in water dissolves in a weakly basic solution, and this solution and a freeze-dried product of this solution were found, thereby leading to completion of the present invention.

Namely, the present invention is a process for producing a chitosan-containing composition comprising the steps of: A) adding an acid to chitosan to dissolve it, thereby preparing an acidic chitosan aqueous solution; B) mixing an acidic polymer compound into the acidic chitosan aqueous solution, and then removing a water-soluble salt from a water-insoluble salt of the chitosan and acidic polymer formed as a result of this mixing; and C) dissolving the water-insoluble salt of chitosan and the acidic polymer compound in a weakly basic solution either directly or after drying to a solid to obtain a chitosan-containing composition in the form of an aqueous solution or gel, and as necessary, comprising a step of obtaining a chitosan- containing composition in the form of a dry powder by drying the resulting chitosan-containing composition in the form of an aqueous solution or gel; and, a chitosan-containing composition obtained according to this production process in the form of any of an aqueous solution, gel or dry powder thereof containing chitosan, an acidic polymer compound and a carbonate, wherein the pH when in the form of an aqueous solution is within the range of 7.0 to 9.5.

The chitosan-containing composition of the present invention is characterized by having a pH within the range of 7.0 to 9.5 when in the form of an aqueous solution, and the pH is preferably within the range of 7.2 to 9. 2 and more preferably within the range of 7.3 to 9.1. If this pH is within the range of 7.0 to 9.5, there is little precipitation of chitosan, thereby making this preferable.

In a preferred embodiment of the present invention, the chitosan-containing composition has high transparency such that when placed in a quartz cell having an optical path length of 10 mm in the state of an aqueous solution having a chitosan concentration of 0.2 to 2% by weight and transmittance is measured at a wavelength of 600 nm, transmittance corrected to a chitosan concentration of 1% is 10% or more, preferably 20% or more, and more preferably 40% or more. If the transmittance in an aqueous solution under the above conditions is 10% or more, there is no sensation of turbidity, thereby making this preferable.

In step A of the production process of the present invention, chitosan is dissolved by adding acid thereto to prepare an acidic chitosan aqueous solution. The method used to dissolve the chitosan may consist of first adding water to the chitosan followed by adding acid or an acidic solution, or directly adding an acidic solution to the chitosan. In addition, a water-soluble salt of chitosan may be dissolved in water. The temperature during this dissolution may be varied from 0 to 100°C, stirring may be carried out as necessary, and dissolution is preferably continued until the solution becomes clear. In addition, a salt such as sodium chloride may also be added to the solution as necessary.

The chitosan used in the present invention is a deacetylation product of chitin contained mainly in the shells of crustaceans such as crabs and shrimp, is a polysaccharide having amino groups, and is a known substance. Examples of commercially available chitosan products include products known by the trade names of "Koyo Chitosan FL-80", "Koyo Chitosan FM-40", "Koyo Chitosan FM-80", "Koyo Chitosan FH-80" and "Koyo Chitosan FM-200" manufactured by Koyo Chemical Co., Ltd., and products known by the trade names of "Chitosan 5", "Chitosan 50", "Chitosan 500" and "Chitosan 1000" manufactured by Wako Pure Chemical Industries, Ltd, and these may be used alone or two or more types can be used in combination. In addition, examples of commercially available water-soluble salts include a product known by the trade name "Koyo Chitosan FLA-40" manufactured by Koyo Chemical Co., Ltd.

In the step A, the acid used to dissolve the chitosan is selected from the group consisting of organic acids such as acetic acid, formic acid, lactic acid, malic acid, citric acid or adipic acid, and inorganic acids such as hydrochloric acid or sulfuric acid, and one of these can be used alone or two or more types can be used in combination either directly or after putting into solution.

In step B of the production process of the present invention, the acidic polymer compound solution added to the acidic chitosan aqueous solution preferably uses that in which the acidic polymer compound is dissolved by adding water thereto. The temperature at this time is within the range of 0 to 100°C, the temperature may be varied, stirring may be carried out as necessary, and dissolution is preferably continued until the solution becomes clear. In addition, a salt such as sodium chloride may be added to the solution as necessary. The amount of this acidic polymer compound is preferably an amount sufficient for causing the chitosan in the acidic chitosan aqueous solution to precipitate in the form of an insoluble salt as much as possible.

Examples of the acidic polymer compound used in the present invention include compounds having a molecular weight of 1000 or more and preferably 5000 or more that contain one or more sulfate groups, carboxyl groups and sodium, potassium, calcium or ammonium salts thereof. Specific examples of these compounds include one type or two or more types selected from the group consisting of carrageenan, sodium chondroitin sulfate, sodium dextran sulfate, sodium alginate and polyacrylic acid.

The carrageenan used as an acidic polymer compound in the present invention is a known substance obtained from a raw material seaweed (red seaweed) such as *Eucheuma cottonii* or *Eucheuma spinosum,* and although each of the types of kappa, lambda and iota are known according to the structure thereof, any of these types may be used or a mixture of two or more types may be used. Examples of commercially available products include "CP Gum FA" (trade name) manufactured by Dainippon Sumitomo Pharma Co., Ltd., and "Carrageenan PA-1", "Carrageenan PA-2", "Carrageenan PA-3", "Carrageenan CS-1", "Carrageenan CS-2" and "Carrageenan CS-3" (trade names) manufactured by Ina Food Industry Co., Ltd., and these may be used alone or two or more types can be used in combination.

The sodium chondroitin sulfate used as an acidic polymer compound in the present invention is a known substance obtained from a raw material cartilage of shark, squid and the like, and examples of commercially available products include "Sodium Chondroitin Sulfate" (trade name) manufactured by Seikagaku Corp., and "Chondroitin Q" (trade name) manufactured by Q.P. Corp.

The polyacrylic acid used as an acidic polymer compound in the present invention is a synthetic polymer, and is a known substance also referred to as carboxyvinyl polymer. Examples of commercially available products include "Carbopol 980", "Carbopol 981", "Carbopol 2984", "Carbopol 5984" and "Carbopol Ultrez 10" (trade names) manufactured by Nikko Chemicals Co., Ltd., and one of these may be used alone or two or more types can be used in combination.

In the step B, in the case of mixing an acidic chitosan aqueous solution and an acidic polymer compound solution, the acidic chitosan aqueous solution may be added to the acidic polymer compound solution, or the acidic polymer compound solution may be added to the acidic chitosan aqueous solution. The temperature at this time is 0 to 100°C, the temperature may be varied, and stirring may be carried out as necessary. In addition, a salt such as sodium chloride may be added to the solution as necessary. The mixed solution is stirred or allowed to stand undisturbed as necessary to form an insoluble salt of chitosan and the acidic polymer compound. The temperature at this time is within the range of 0 to 100°C, and the temperature may be varied.

After having adequately formed the insoluble salt of chitosan and the acidic polymer compound (to be referred to as a solid), the solid is separated from water-soluble salt dissolved in the liquid phase by separating the solid from the liquid phase. This separation treatment can be carried out using any treatment method such as filtration, centrifugal separation, pressing or dialysis. In addition, the water-soluble salt may be further separated from the solid (washing of the solid) by a method such as filtration, centrifugal separation or pressing after repeatedly adding water to the separated solid.

A wet solid is obtained by the solid separation procedure described above. This wet solid may be used directly as a material of the following step C without particularly having to dehydrate or dry. In addition, the wet solid may also be crushed as necessary. This wet solid may be dried to a dry solid by air drying, freeze-drying, vacuum drying or spray drying and the like, and the dried product may further be crushed as necessary.

In step C in the production process of the present invention, the solid in various states (wet or dry) obtained in step B is dissolved in a weakly basic solution to prepare a chitosan-containing composition in the form of a neutral to weakly alkaline, highly transparent aqueous solution or gel having a pH within the range of 7.0 to 9.5. The method used to dissolve the solid in the weakly basic solution may consist of adding the weakly basic solution to the solid, adding the solid to the weakly basic solution, or mixing the solid with water followed by adding the weakly basic solution salt. Stirring may be carried out at this time as necessary.

An aqueous solution containing one type or two or more types selected from the group consisting of sodium bicarbonate, ammonium bicarbonate and ammonium carbonate is preferable for the weakly basic solution used in the step C. It is preferable to add a suitable amount of this weakly basic solution while measuring the pH of the mixture so that the pH of the aqueous solution or gel obtained after dissolving the solid is within the aforementioned range.

According to the present invention as described above, a chitosan-containing composition containing chitosan and in the form of a highly transparent aqueous solution or gel having a neutral to weakly alkaline pH when in the form of an aqueous solution can be provided, and as a result thereof, a product containing chitosan, having high transparency, and having a superior sensory properties free of a sour taste can be provided for use in fields such as foods, beverages, cosmetics and pharmaceuticals.
In addition, the present invention provides a means for incorporating chitosan in compounds having low stability under acidic conditions in fields such as foods, beverages, cosmetics and pharmaceuticals.
In addition, the production process of a chitosan-containing composition of the present invention is able to easily provide the aforementioned industrially useful chitosan-containing composition as described above.

In addition, in the present invention, a chitosan-containing composition can be obtained in the form of a dry powder by drying the chitosan-containing composition in the form of an aqueous solution or gel obtained in the aforementioned step C. A dry powder of a chitosan-containing composition obtained thereby can be used as a gelling additive in the production of pharmaceuticals.

Although the following provides a more detailed explanation of the present invention through examples thereof, the following examples are merely exemplary of the present invention, and the present invention is not limited to only these examples.

### [Example 1]

2.7 g of chitosan (trade name "Chitosan 500", Wako Pure Chemical Industries, Ltd.) were added to 97 mL of purified water and suspended therein followed by the addition of 3 mL of acetic acid and 5.44 g of sodium chloride and uniformly dissolving by stirring for 15 minutes at 90°C to prepare an acidic chitosan aqueous solution.
Separate from the above, 5.44 g of sodium chloride and 4.3 g of carrageenan (trade name "κ-Carrageenan", Wako Pure Chemical Industries, Ltd.) were added to 100 mL of purified water followed by stirring for 15 minutes at 90°C to prepare a carrageenan solution.
The two solutions were then mixed followed by additionally stirring for 15 minutes at 90°C. Next, the mixture was chilled in a refrigerator overnight after allowing to cool.
Next, the solid that formed was pressed, the water-soluble salt was separated, and 300 mL of water were further added followed by pressing to further separate the water-soluble salt.
Half of the solid separated from the water-soluble salt was dissolved in 250 mL of a 0.10 mol/L aqueous sodium bicarbonate solution to prepare a transparent, highly viscous solution. The pH of this solution was 9.03. In addition, as a result of placing this solution in a quartz cell having an optical path length of 10 mm and measuring transmittance at a wavelength of 600 nm, the transmittance corrected to a chitosan concentration of 1% was 68.68%.

### [Example 2]

2.7 g of chitosan (trade name "Chitosan 500", Wako Pure Chemical Industries, Ltd.) were added to 97 mL of purified water and suspended therein followed by the addition of 3 mL of acetic acid and 5.44 g of sodium chloride and uniformly dissolving by stirring for 15 minutes at 90°C to prepare an acidic chitosan aqueous solution.
Separate from the above, 5.44 g of sodium chloride and 4.3 g of carrageenan (trade name "κ-Carrageenan", Wako Pure Chemical Industries, Ltd.) were added to 100 mL of purified water followed by stirring for 15 minutes at 90°C to prepare a carrageenan solution.
The two solutions were then mixed followed by additionally stirring for 15 minutes at 90°C. Next, the mixture was chilled in a refrigerator overnight after allowing to cool.
Next, the solid that formed was pressed, the water-soluble salt was separated, and 300 mL of purified water were further added followed by pressing to further separate the water-soluble salt.
Half of the solid separated from the water-soluble salt was freeze-dried and then crushed to obtain a solid dry powder. 1.4 mL of a 0.2 mol/L sodium bicarbonate solution and 0.35 mL of purified water were then added to 0.017 g of this solid dry powder to obtain a homogeneous, transparent and viscous solution having a pH of 8.19. As a result of placing this solution in a quartz cell having an optical path length of 10 mm and measuring transmittance at a wavelength of 600 nm, the transmittance corrected to a chitosan concentration of 1% was 15.00%.

### [Example 3]

A nearly transparent, viscous solution having a pH of 9. 12 was obtained by adding 2 mL of a 0.2 mol/L ammonium carbonate solution and 3 mL of purified water to 0.05 g of the solid dry powder prepared in Example 2.
This solution had nearly the same appearance as that of Example 2.

### [Example 4]

0.27 g of chitosan (trade name "Koyo Chitosan FL-80", Koyo Chemical Co., Ltd.) were added to 10 mL of purified water and suspended therein followed by the addition of 0.34 g of citric acid and uniformly dissolving by stirring for 15 minutes at 90°C to prepare an acidic chitosan aqueous solution.
Separate from the above, 0.43 g of κ-carrageenan (trade name "κ-Carrageenan", Wako Pure Chemical Industries, Ltd.) were added to 10 mL of purified water followed by stirring for 15 minutes at 90°C to prepare a carrageenan solution.
The two solutions were then mixed followed by additionally stirring for 15 minutes at 90°C. Next, the mixture was chilled in a refrigerator overnight after allowing to cool.
Next, the gel-like solid that formed was pressed, the water-soluble salt was separated, and 30 mL of water were further added followed by pressing to further separate the water-soluble salt. The resulting solid was freeze-dried and then crushed to obtain a solid dry powder.
4 mL of a 0.2 mol/L sodium bicarbonate solution and 1 mL of purified water were added to 0.05 g of the solid dry powder to obtain a homogeneous, transparent solution having a pH of 7.40. This solution had nearly the same appearance as that of Example 2.

### [Example 5]

A nearly transparent solution having a pH of 7.67 was obtained by adding 5 ml of a 0.2 mol/L ammonium bicarbonate solution to 0.05 g of the solid dry powder prepared in Example 4. This solution had nearly the same appearance as that of Example 2.

### [Example 6]

0.27 g of chitosan (trade name "Koyo Chitosan FM-40", Koyo Chemical Co., Ltd.) were added to 10 mL of purified water and suspended therein followed by the addition of 2 mL of 1 mol/L hydrochloric acid and uniformly dissolving by stirring for 15 minutes at 90°C to prepare an acidic chitosan aqueous solution.
Separate from the above, 0. 43 g of τ-carrageenan (trade name "τ-Carrageenan", Tokyo Chemical Industry Co., Ltd.) were added to 10 mL of purified water followed by stirring for 15 minutes at 90°C to prepare a carrageenan solution.
The two solutions were then mixed followed by additionally stirring for 15 minutes at 90°C. Next, the mixture was chilled in a refrigerator overnight after allowing to cool.
Next, the gel-like solid that formed was pressed, the water-soluble salt was separated, and 30 mL of purified water were further added followed by pressing to further separate the water-soluble salt. The resulting solid was freeze-dried and then crushed to obtain a solid dry powder.
5 mL of a 0.2 mol/L sodium bicarbonate solution were added to 0.05 g of the solid dry powder to obtain a homogeneous, transparent solution having a pH of 7.36. This solution had nearly the same appearance as that of Example 2.

### [Example 7]

0.13 g of chitosan (trade name "Koyo Chitosan FL-80", Koyo Chemical Co., Ltd.) were added to 5 mL of purified water and suspended therein followed by the addition of 0.17 g of citric acid and uniformly dissolving by stirring for 15 minutes at room temperature to prepare an acidic chitosan aqueous solution.
Separate from the above, 0.21 g of sodium chondroitin sulfate (trade name "Sodium Chondroitin Sulfate", Seikagaku Corp.) were added to 5 mL of purified water followed by stirring for 15 minutes at room temperature.
The two solutions were then mixed followed by additionally stirring for 15 minutes. Next, the solid that formed was centrifugally separated and the supernatant was discarded followed by the addition of 20 ml of purified water, washing and repeating desalting three times.
Purified water was then added to the washed solid to bring to a total volume of 20 mL followed by dissolving the solid by adding 0.5 g of sodium bicarbonate. The resulting solution was freeze-dried to obtain 0.6855 g of a chitosan-containing composition in a dry state.
Moreover, 10 mL of purified water were added thereto to obtain a transparent solution. This solution had nearly the same appearance as that of Example 2.

### [Example 8]

0.13 g of chitosan (trade name "Koyo Chitosan FL-80", Koyo Chemical Co., Ltd.) were added to 5 mL of purified water and suspended therein followed by the addition of 0.17 g of citric acid and uniformly dissolving by stirring for 15 minutes to prepare an acidic chitosan aqueous solution.
Separate from the above, 0.21 g of sodium alginate (trade name "Sodium Alginate 80-120", Wako Pure Chemical Industries, Ltd.) were added to 10 mL of purified water followed by stirring for 15 minutes.
The two solutions were then mixed followed by additionally stirring for 15 minutes. Next, 1 g of sodium chloride was added thereto followed by additionally stirring for 1 hour. Next, the solid that formed was centrifugally separated and the supernatant was discarded followed by the addition of 20 ml of purified water, washing and repeating desalting three times.
Purified water was then added to the washed solid to bring to a total volume of 20 mL followed by dissolving the solid by adding 0.5 g of sodium bicarbonate. The resulting solution was freeze-dried to obtain 0.5298 g of a chitosan-containing composition in a dry state.
Moreover, 10 mL of purified water were added thereto to obtain a transparent solution. This solution had nearly the same appearance as that of Example 2.

### [Example 9]

0.13 g of chitosan (trade name "Koyo Chitosan FL-80", Koyo Chemical Co., Ltd.) were added to 5 mL of purified water and suspended therein followed by the addition of 0.17 g of citric acid and uniformly dissolving by stirring for 15 minutes to prepare an acidic chitosan aqueous solution.
Separate from the above, 0.21 g of sodium dextran sulfate (trade name "Sodium Dextran Sulfate 5000", Wako Pure Chemical Industries, Ltd.) were added to 5 mL of purified water followed by stirring for 15 minutes.
The two solutions were then mixed followed by the addition of 1 g of sodium chloride and additionally stirring for 3 hours.

The solid that formed was centrifugally separated and the supernatant was discarded followed by the addition of 10 ml of purified water, washing and repeating desalting three times.
10 mL of purified water were then added to the washed solid to suspend therein followed by freeze-drying to obtain 0.160g of a dry solid.
Next, the resulting dry solid was dissolved by adding 10 mL of purified water and 0.5 g of sodium bicarbonate to obtain a transparent, somewhat viscous solution having a pH of 7.89. As a result of placing this solution in a quartz cell having an optical path length of 10 mm and measuring transmittance at a wavelength of 600 nm, the transmittance corrected to a chitosan concentration of 1% was 48.08%.

### [Comparative Example 1]

0.27 g of chitosan (trade name "Koyo Chitosan FL-80", Koyo Chemical Co., Ltd.) were added to 10 mL of purified water and suspended therein followed by the addition of 0.34 g of citric acid and uniformly dissolving by stirring for 15 minutes at 90°C to prepare an acidic chitosan aqueous solution. The pH of this acidic chitosan aqueous solution was 2.79.

A 1 mol/L sodium hydroxide solution was then dropped into this acidic chitosan aqueous solution to neutralize while monitoring the pH of the solution.
When the pH of the solution reached 3.79 or more, chitosan precipitated and the solution became turbid. When the pH of the solution reached 7.45, dropping of sodium hydroxide solution was discontinued, and as a result of placing the turbid solution in a quartz cell having an optical path length of 10 mm and measuring transmittance at a wavelength of 600 nm, the transmittance of the solution was 0.5% or less.
Since the resulting turbid solution had a high concentration of water-soluble salt contained therein and had a poor appearance, practical application thereof in food applications such as beverage production was difficult.

## Claims

1. A chitosan-containing composition in the form of an aqueous solution, gel or dry powder and containing chitosan, an acidic polymer compound and a carbonate or bicarbonate, wherein the pH when in the form of an aqueous solution is within the range of 7.0 to 9.5.

2. The chitosan-containing composition according to claim 1, wherein the acidic polymer compound is one type or two or more types selected from the group consisting of carrageenan, sodium chondroitin sulfate, sodium dextran sulfate, sodium alginate and polyacrylic acid.

3. The chitosan-containing composition according to claim 1 or 2, wherein the carbonate or bicarbonate is one type or two or more types selected from the group consisting of sodium bicarbonate, ammonium bicarbonate and ammonium carbonate.

4. The chitosan-containing composition according to any of claims 1 to 3, wherein the chitosan-containing composition is in the form of an aqueous solution.

5. The chitosan-containing composition according to any of claims 1 to 3, wherein the chitosan-containing composition is in the form of a gel.

6. The chitosan-containing composition according to any of claims 1 to 3, wherein the chitosan-containing composition is in the form of a dry powder.

7. A process for producing a chitosan-containing composition comprising the steps of:
A) adding acid to chitosan to dissolve it thereby preparing an acidic chitosan aqueous solution;
B) mixing an acidic polymer compound into the acidic chitosan aqueous solution, thereby forming a water-insoluble salt of the chitosan and the acidic polymer compound; and removing water-soluble salt from the salt, thereby forming a solid; and
C) dissolving the solid in a weakly basic solution either directly or after drying to a solid, thereby obtaining a chitosan-containing composition in the form of an aqueous solution or gel.

8. The process for producing a chitosan-containing composition according to claim 7, further comprising a step of drying the chitosan-containing composition in the form of an aqueous solution or gel obtained in the step C, thereby obtaining a chitosan-containing composition in the form of a dry powder.

9. The process for producing a chitosan-containing composition according to claim 7 or 8, wherein the acidic polymer compound is one type or two or more types selected from the group consisting of carrageenan, sodium chondroitin sulfate, sodium dextran sulfate, sodium alginate and polyacrylic acid.

10. The process for producing a chitosan-containing composition according to any one of the claims 7 to 9, wherein the weakly basic solution is an aqueous solution containing one type or two or more types selected from the group consisting of sodium bicarbonate, ammonium bicarbonate and ammonium carbonate.
